# EUROPEAN PATENT SPECIFICATION

(11) **EP 1 616 005 B1**
(45) Date of publication and mention of the grant of the patent: **04.03.2009**
(21) Application number: 03717817.5
(22) Date of filing: 22.04.2003
(51) Int. Cl.: C12N 9/96, C12N 1/04, A61K 31/70, A61K 47/26

(54) **MANNOSYL(1-2)GLYCOSYL(1-2)GLYCERATE FOR THE STABILISATION AND PRESERVATION OF BIOMATERIALS**
MANNOSYL(1-2)GLYCOSYL(1-2)GLYCERAT ZUR STABILISIERUNG UND KONSERVIERUNG VON BIOMATERIALIEN
MANNOSYL(1-2)GLYCOSYL(1-2)GLYCERATE PERMETTANT DE STABILISER ET DE CONSERVER DES BIOMATERIAUX

(43) Date of publication of application: 18.01.2006
(73) Proprietor: Instituto de Biologia Experimental e Tecnologia (IBET), 2781-901 Oeiras (PT)
(72) Inventor: SANTOS, Helena, 2775-713 Carcavelos (PT); ANTÓNIO, Pedro Miguel Lamosa, Lisboa (PT); JORGE, Carla, 2800 Almada (PT); DA COSTA, Milton Simoes, 3770 Oliveira do Bairro (PT)
(74) Representative: Ferreira, Maria Silvina
(86) International application number: PCT/PT2003/000006
(87) International publication number: WO 2004/094631

(56) References cited:
- EP-A- 0 816 509
- EP-A- 0 965 268
- WO-A-01/58446
- WO-A-01/76572
- BORGES N ET AL.: "Comparative study of the thermostabilizing properties of mannosylglycerate and other compatible solutes on model enzymes." EXTREMOPHILES, vol. 6, no. 3, June 2002 (2002-06), pages 209-216, XP002253694 ISSN: 1431-0651 cited in the application
- RAMOS A ET AL.: "Stabilization of enzymes against thermal stress and freeze-drying by mannosylglycerate" APPLIED AND ENVIRONMENTAL MICROBIOLOGY, vol. 63, no. 10, October 1997 (1997-10), pages 4020-4025, XP002114723 ISSN: 0099-2240 cited in the application
- MARTINS L O ET AL.: "New compatible solutes related to Di-myo-inositol-phosphate in members of the order Thermotogales." JOURNAL OF BACTERIOLOGY, vol. 178, no. 19, October 1996 (1996-10), pages 5644-5651, XP002253693 ISSN: 0021-9193
- CÁNOVAS D ET AL.: "Role of Ngamma-acetyldiaminobutyrate as an enzyme stabilizer and an intermediate in the biosynthesis of hydroxyectoine." APPLIED AND ENVIRONMENTAL MICROBIOLOGY, vol. 65, no. 9, September 1999 (1999-09), pages 3774-3779, XP002253695 ISSN: 0099-2240
- EMPADINHAS N ET AL.: "Pathway for the synthesis of mannosylglycerate in the hyperthermophilic archaeon Pyrococcus horikoshii. Biochemical and genetic characterization of key enzymes." JOURNAL OF BIOLOGICAL CHEMISTRY, vol. 276, no. 47, 23 November 2001 (2001-11-23), pages 43580-43588, XP002253696 ISSN: 0021-9258
- SANTOS H ET AL.: "Compatible solutes of organisms that live in hot saline environments." ENVIRONMENTAL MICROBIOLOGY, vol. 4, no. 9, September 2002 (2002-09), pages 501-509, XP002253697 ISSN: 1462-2912
- SANTOS H ET AL.: "Organic solutes from thermophiles and hyperthermophiles" METHODS IN ENZYMOLOGY, ACADEMIC PRESS INC, SAN DIEGO, CA, US, vol. 334, 2001, pages 302-315, XP008021120 ISSN: 0076-6879 cited in the application
- DA COSTA M S ET AL.: "An overview of the role and diversity of compatible solutes in bacteria and archaea" ADVANCES IN BIOCHEMICAL ENGINEERING, BIOTECHNOLOGY, SPRINGER, BERLIN, DE, vol. 61, 1998, pages 117-153, XP002099622 ISSN: 0724-6145

## Description

### FIELD OF THE INVENTION

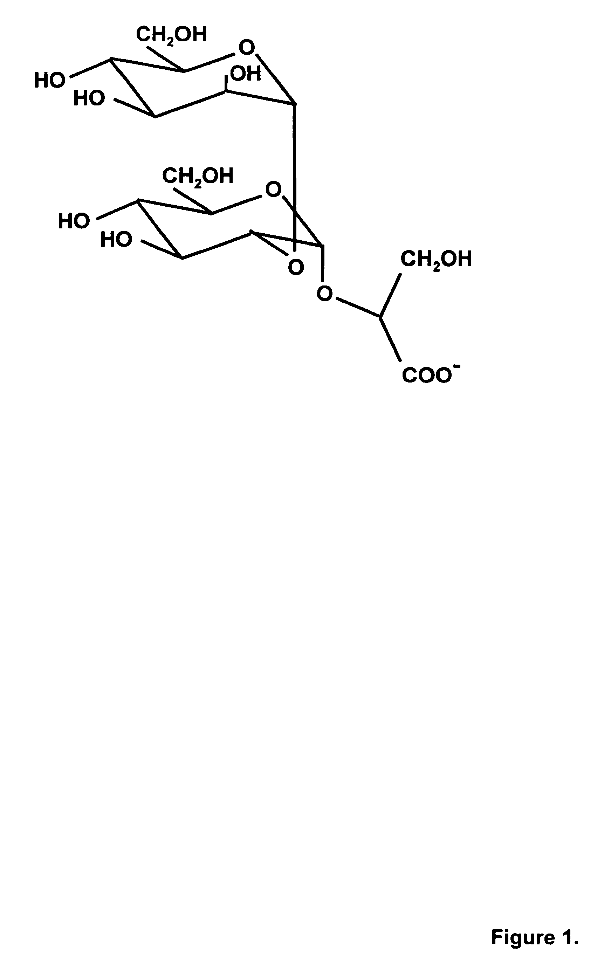

The present invention concerns the use of mannosyl(1-2)glucosyl(1-2)glycerate, in any of its possible stereoisomeric forms, alone or as a constituent of formulations to protect and/or stabilize enzymes or other cellular components and biomaterials against general stress, namely caused by heat, high osmolarity, free-radicals, desiccation, freeze-drying, and repetitive use. Said compounds obey to the general formula depicted in Figure 1.

### DESCRIPTION OF THE INVENTION AND STATE OF THE ART

The accumulation of low-molecular mass organic solutes such as, trehalose, polyols or ectoines, is a prerequisite for osmotic adjustment of many mesophilic microorganisms. However, very unusual solutes namely, *di-myo-inositol phosphate,* di-mannosyl-di-myo-inositvl-phosphate, diglycerol phosphate, mannosylglycerate, and mannosylglyceramide, have been identified in thermophilic and hyperthermophilic microorganisms and the intracellular content of these solutes increases in response to stress conditions, such as high osmolarity or high temperature.

Mannosylglycerate and diglycerol phosphate have been studied to a greater extent and have been shown to protect enzymes and proteins *in vitro* better than compatible solutes from mesophiies [1-3]. Moreover, the application of compatible solutes from thermophilic or hyperthermophilic organisms, as stabilising agents of biomaterials, has been disclosed in several patent applications [4-6].

We have discovered a novel compatible solute in the thermophilic bacterium *Petrotoga miotherma,* an organism that grows optimally at 55°C, but is able to grow as high as 65°C. When subjected to salt stress this organism accumulates large amounts (above 1 µmol/mg of protein) of a novel di-sugar compound. After extraction, purification, and full spectroscopic characterization, by Nuclear Magnetic Resonance, we have determined the molecular structure of this, to date, unknown compound as α(1-2)mannopyranosyl-α(1-2)glucopyranosyl-glycerate.
It is interesting to note that the molecular structure of this compatible solute comprises the two moieties (mannosyl and glyceryl) present in mannosylglycerate, a solute widely distributed among thermophiles and hyperthermophiles [7]. In addition, there is a glucosyl moiety linking the mannosyl and glyceryl moieties.

Mannosylglycerate is a well-known biostabiliser of thermophilic origin, whose industrial application is protected under a European patent application [4]. The thermophilic origin of the novel solute combined with the structural resemblance to mannosylglycerate leads us to propose that this novel solute has stabilising properties as good or superior to those already demonstrated for mannosylglycerate. In this respect, it will serve as a stabiliser in various commercial, industrial, medical, pharmaceutical, diagnostic, cosmetic, or academic research applications.

The enhanced protein stability rendered by certain low-molecular mass organic solutes allows enzymes to function under more severe conditions of temperature, pressure, ionic strength, pH, presence of detergents or organic solvents. One of the priorities of modern biotechnology is to obtain stable enzymes or agents that stabilise those enzymes against thermal or chemical denaturation. The ability of some compatible solutes to stabilise enzymes is, therefore, of great importance to modem biotechnology. This point is obviously extended to all proteins that are used or can be used in processes where their stability is an issue, since all proteins, either with or without enzymatic activity, share the same overall basic elements of structure and may be protected against denaturation or inactivation through the same general mechanisms or processes.
II must also be stressed that compatible solutes protect proteins, cell membranes, lipossomes, and cells from the deleterious effects of desiccation, and possess strong moistening properties. The preservation of desiccated or lyophilized cell components and biomaterials has many applications in medicine, pharmaceutical industry, cosmetic industry, food industry, and scientific research. In spite of the great importance of desiccation and freezing in the conservation of biological samples, denaturation of proteins or a decrease of the viable count of cultures inevitably takes place during utilization, and could be prevented or diminished by the use of low molecular mass stabilisers. Also, the stability of nucleic acid molecules, like DNA, or RNA, can be improved by the addition of compatible solutes from hyperthermophiles, as described for ectoines [8], and their use in several applications in medicine, pharmaceutical industry, or scientific research can be envisioned.

### BRIEF DESCRIPTION OF THE DRAWING

**Figure 1****.**
   Depicts the generic chemical structure of mannosyl(1-2)glucosyl(-2)glycerate in all its possible stereoisomeric forms. The figure is intended to represent both mannosyl and glucosyl moieties either in the α or in the β configuration.

### REFERENCES

[1] Ramos, A., N. D. H. Raven, R. J. Sharp, 5. Bartolucci, M. Rossi, R. Cannio, J. Lebbink, J. van der Oost, W. M. de Vos and H. Santos. 1997. Stabilisation of enzymes against thermal stress and freeze-drying by mannosylglycerate. Appl. Environ. Microbiol. 63:4020-4025.
[2] Borges, N., A. Ramos, N. O. H. Raven, R. J. Sharp, and H. Santos. 2002. Comparative study of the thermostabilising properties of mannosylglycerate and other compatible solutes on model enzymes. Extremophiles 6:209-216.
[3] Lamosa, P., A. Burke, R. Peist, R. Huber, M. Liu, G. Silva, C. Rodrigues-Pousada, J. LeGall, C. Maycock, and H. Santos. 2000. Thermostabilisation of proteins by diglycerol phosphate, a new compatible solute from the hyperthermophile Archaeoglobus fulgidus. Appl. Environ. Microbiol. 66:1974-1979.
[4] Santos, H., A. Ramos, M. S. da Costa. Thermostabilisation, osmoprotection, and protection against desiccation of enzymes, cell components and cells by mannosylglycerate. EP-A-0 816 509.
[5] Santos, H., P. Lamosa, C. Maycock, and A. Burke, 1999. Thermostabilisation, osmoprotection, and protection against desiccation of enzymes, cell components and cells by di-glycerol-phosphate. EP-A-O 965 268.
[6] Santos, H., L O. Martins, L. 5. Carreto, and M. 5. da Costa. 1996. Utilização de fosfato de di-manosil-di-mio-inositol e de fosfato de 1,3'- di-mio-inositol na termoestabilisação, osmoprotecção e protecção contra a desidratação de componentes celulares e células. Portuguese patent no. 101813.
[7] Santos. H., and M. 5. da Costa. 2009. Organic solutes from thermophiles and hyperthermophiles. Methods Enzymol. 334:302-315.
[8] Malin G, Iakobashvili R, and Lapidot A (1999) Effect of tetrahydropyrimidine derivatives on protein-nucleic acids interaction. Type II restriction endonucleases as a model system, J. Biol. Chem. 274:6920-6929.

## Claims

1. The use of mannosyl(1-2)glucosyl(1-2)glycerate wherein the compound is in any of its possible stereoisomeric forms, alone or as a constituent in a suitable formulation to protect and/or stabilise enzymes or other materials of biological origin against stress caused by heat, high osmolarity, free-radicals, desiccation, freeze-drying, and/or repetitive use.

2. The use of mannosyl(1-2)glucosyl(1-2)glycerate according to claim 1 wherein the glucosyl and mannosyl residues are in the α or β configurations.

3. The use of mannosyl(1-2)glucosyl(1-2)glycerate according to claims 1 and 2 wherein the glycerate residue is in the D or L configuration.

4. The use of mannosyl(1-2)glucosyl(1-2)glycerate or mixtures thereof according to claims 1 to 3 to protect enzymes or other proteins against temperature denaturation induced by purification, transport and/or storage.

5. The use of mannosyl(1-2)glucosyl(1-2)glycerate or mixtures thereof according to claims 1 to 3 as a protector of the activity of polymerase chain reaction (PCR) enzymes for clinical, biological and industrial purposes during the storage as well as the high-temperature recycling of the enzymes.

6. The use of mannosyl(1-2)glucosyl(1-2)glycerate or mixtures thereof according to claims 1 to 3 as a stabiliser of enzymes or other proteins during lyophilisation, desiccation or freeze-drying and storage.

7. The use of mannosyl(1-2)glucosyl(1-2)glycerate or mixtures thereof according to claims 1 to 3 as a stabilising agent during the manufacture, storage and assays using test kit enzymes, for commercial, biological and industrial purposes.

8. The use of mannosyl(1-2)glucosyl(1-2)glycerate or mixtures thereof according to claims 1 to 3 to stabilise enzymes or other proteins during their routine use for commercial, biological and industrial purposes.

9. The use of mannosyl(1-2)glucosyl(1-2)glycerate or mixtures thereof according to claims 1 to 3 for the protection or stabilisation of antibodies for commercial, biological, research and industrial purposes.

10. The use of mannosyl(1-2)glucosyl(1-2)glycerate or mixtures thereof according to claims 1 to 3 for the protection or stabilisation of vaccines of proteic or nonproteic nature for commercial or industrial purposes.

11. The use of mannosyl(1-2)glucosyl(1-2)glycerate or mixtures thereof according to claims 1 to 3 for the protection of materials of biological origin against stress such as desiccation and lyophilisation of cell membranes, liposomes, liposome-containing cosmetics or lipid related substances.

12. The use of mannosy(1-2)glucosyl(1-2)glycerate or mixtures thereof according to claims 1 to 3 for the structural protection or stabilisation of DNA or RNA molecules.

13. The use of mannosyl(1-2)glucosyl(1-2)glycerate or mixtures thereof according to claims 1 to 3 for the protection, stabilisation or improved performance of DNA or RNA microarrays.

14. The use of mannosyl(1-2)glucosyl(1-2)glycerate or mixtures thereof according to claims 1 to 3 as an additive to cosmetics to improve moistening properties, stabilise compounds or liposomes or as a suppressor of free radicals.

15. The use of mannosyl(1-2)glucosyl(1-2)glycerate or mixtures thereof according to claims 1 to 3 for protection against damage caused by lyophilisation, desiccation, temperature stress, and freezing to microbial cells.

16. The use of mannosyl(1-2)glucosyl(1-2)glycerate or mixtures thereof according to claims 1 and 4 to 15 wherein the compound is a constituent in a suitable formulation to protect and/or stabilise enzymes or other cellular components and biomaterials in commercial, biological, research, and industrial purposes.

## Patentansprüche

1. Verwendung von Mannosyl(1-2)glykosyl(1-2)glyzerat, in welcher die Verbindung sich in irgendeine ihren möglischen stereoisomeren Formen befindet, entweder allein oder als einem Bestandteil einer geeigneten Formulierung um Enzyme und andere Stöffe aus biologischen Quellen stammend, gegen Stress durch Wärme, höhe Osmolarität, Freieradikale, Troknung, Gefriertrocknung und/oder wiederholte Anwendung verursachte, zu schützen.

2. Verwendung von Mannosyl(1-2)glykosyl(1-2)glyzerat nach Anspruch 1, in welcher sich die Mannosyl- und Glykosylreste in die α- oder β-Gestaltung befinden.

3. Verwendung von Mannosyl(1-2)glykosyl(1-2)glyzerat nach Ansprüche 1 und 2, in welcher sich der Glyzeratrest in den D- oder L-Gestaltunge befindet.

4. Verwendung von Mannosyl(1-2)glykosyl(1-2)glyzerat oder ihren Mischungen nach Ansprüche 1 bis 3, um Enzyme und andere Proteine gegen Temperaturdenaturierung durch Reinigung, Transport und/oder Lagerung verursacht, zu schützen.

5. Verwendung von Mannosyl(1-2)glykosyl(1-2)glyzerat oder ihren Mischungen nach Ansprüche 1 bis 3, als einem Schutzmittel der Aktivierung des Polymerase-Chain-Reaction (PCR) Enzyme, für klinische, biologische und industrielle Zwecke während der Lagerung, sowie für das höhe Temperatur Recycling der Enzyme.

6. Verwendung von Mannosyl(1-2)glykosyl(1-2)glyzerat oder ihren Mischungen nach Ansprüche 1 bis 3, als einem Stabilisator für Enzyme oder andere Proteine während der Gefriertrocknung, Troknung oder Gefriertrocknung und Lagerung.

7. Verwendung von Mannosyl(1-2)glykosyl(1-2)glyzerat oder ihren Mischungen nach Ansprüche 1 bis 3, als einem Stabilisator während der Herstellung, Lagerung und Teste durch Verwendung von Test-Kits-Enzyme, für handels, biologische und industrielle Zwecke.

8. Verwendung von Mannosyl(1-2)glykosyl(1-2)glyzerat oder ihren Mischungen nach Ansprüche 1 bis 3, um Enzyme oder andere Proteine, während ihre gewohnheitsmäßige Verwendung für handels, biologische und industrielle Zwecke zu stabilisieren.

9. Verwendung von Mannosyl(1-2)glykosyl(1-2)glyzerat oder ihren Mischungen nach Ansprüche 1 bis 3, um Antikörper für handels, biologische und industrielle Zwecke, sowie für Forschung, zu schützen oder stabilisieren.

10. Verwendung von Mannosyl(1-2)glykosyl(1-2)glyzerat oder ihren Mischungen nach Ansprüche 1 bis 3, um Impfstoffe aus Eiweiß oder nicht Eiweiß Art, für handels und industrielle Zwecke, zu schützen oder stabilisieren.

11. Verwendung von Mannosyl(1-2)glykosyl(1-2)glyzerat oder ihren Mischungen nach Ansprüche 1 bis 3, um Stoffe aus biologische Herkunft, gegen Stress zu schützen oder stabilisieren, wie z. B., Trocknung und Gefriertrocknung aus Zellmembrane, Liposomen, liposomenthältende Hautpflegemittel oder Fettkörperänlische Stoffe.

12. Verwendung von Mannosyl(1-2)glykosyl(1-2)glyzerat oder ihren Mischungen nach Ansprüche 1 bis 3, um DNS oder RNS Moleküle strukturell zu schützen oder zu stabilisieren.

13. Verwendung von Mannosyl(1-2)glykosyl(1-2)glyzerat oder ihren Mischungen nach Ansprüche 1 bis 3, um DNS- oder RNS-Mikroarrays zu schützen oder zu stabilisieren, oder um ihre Bestleistung zu verbessern.

14. Verwendung von Mannosyl(1-2)glykosyl(1-2)glyzerat oder ihren Mischungen nach Ansprüche 1 bis 3, als einem Zusatzmittel für Hautpflegemitteln, um die Wasserstoffaufnahmeeigenschäfte zu verbessern, die Verbindungen und Liposomen zu stabilisieren, oder als einem Mittel für Freiradikale zu unterdrücken.

15. Verwendung von Mannosyl(1-2)glykosyl(1-2)glyzerat oder ihren Mischungen nach Ansprüche 1 bis 3, um die Schaden durch Gefriertrocknung, Tröknung, Temperaturstress und Gefrieren auf mikrobielle Zellen verursacht zu schützen.

16. Verwendung von Mannosyl(1-2)glykosyl(1-2)glyzerat oder ihren Mischungen nach Ansprüche 1 und 4 bis 15, in welche die Verbindug ein Bestandteil einer geeigneten Formulierung darum ist, um Enzyme oder andere Zellbestandteile und biologische Stoffe, für handels, biologische, und industrielle Zwecke, sowie für Forschung, zu schützen und/oder zu satibilisieren.

## Revendications

1. Utilisation de mannosyl(1-2)glucosyl(1-2)glycérat, ce composé étant dans quelqu'une de ses formes stéreoisomériques possibles, seule ou comme composant dans une formulation convenable pour protéger et/ou stabiliser des enzymes ou d'autres matériaux d'origine biologique contre le stress origine par la chaleur, l'haute osmolarité, des radicaux libres, dessiccation, lyophilisation, e/ou l'usage répétitive.

2. Utilisation de mannosyl(1-2)glucosyl(1-2)glycérat selon la revendication 1, les résidus glucosyl et mannosyl étant dans les configurations α ou β.

3. Utilisation de mannosyl(1-2)glucosyl(1-2)glycérat selon les revendications 1 et 2, le résidu glycérat étant dans les configurations D ou L.

4. Utilisation de mannosyl(1-2)glucosyl(1-2)glycérat ou de leurs mélanges, selon les revendications 1 à 3, pour protéger des enzymes ou d'autres protéines contre la dénaturation de la température induite par la purification, le transport e/ou le stockage.

5. Utilisation de mannosyl(1-2)glucosyl(1-2)glycérat ou de leurs mélanges, selon les revendications 1 à 3, en tant qu'un protecteur de l'activité des enzymes de réaction en chaîne de polymérase (PCR) pour des buts cliniques, biologiques et industriels, pendant le stockage, aussi bien que pour le recyclage des enzymes à haute température.

6. Utilisation de mannosyl(1-2)glucosyl(1-2)glycérat ou de leurs mélanges, selon les revendications 1 à 3, en tant qu'un stabilisateur d'enzymes ou d'autres protéines pendant la lyophilisation, la dessication ou le séchage par congélation et le stockage.

7. Utilisation de mannosyl(1-2)glucosyl(1-2)glycérat ou de leurs mélanges, selon les revendications 1 à 3, en tant qu'un stabilisateur pendant la fabrication, le stockage et des essais avec l'utilisation enzymes de kits d'essai, pour des buts commerciaux, biologiques et industriels.

8. Utilisation de mannosyl(1-2)glucosyl(1-2)glycérat ou de leurs mélanges, selon les revendications 1 à 3, pour stabiliser des enzymes ou d'autres protéines pendant leur utilisation par routine pour des buts commerciaux, biologiques et industriels.

9. Utilisation de mannosyl(1-2)glucosyl(1-2)glycérat ou de leurs mélanges, selon les revendications 1 à 3, pour la protection ou la stabilisation d'anticorps pour des buts commerciaux, biologiques, de recherche et industriels.

10. Utilisation de mannosyl(1-2)glucosyl(1-2)glycérat ou de leurs mélanges, selon les revendications 1 à 3, pour la protection ou la stabilisation de vaccines de nature protéique ou non protéique, pour des buts comerciaux ou industriels.

11. Utilisation de mannosyl(1-2)glucosyl(1-2)glycérat ou de leurs mélanges, selon les revendications 1 à 3, pour la protection de matériaux d'origine biologique contre le stress, tel que la dessiccation et la lyophilisation de membranes cellulaires, des liposomes, des produits cosmétiques contenant des liposomes ou des matières rélatives aux lipides.

12. Utilisation de mannosyl(1-2)glucosyl(1-2)glycérat ou de leurs mélanges, selon les revendications 1 à 3, pour la protection structurelle ou la stabilisation de molécules de ADN ou de ARN.

13. Utilisation de mannosyl(1-2)glucosyl(1-2)glycérat ou de leurs mélanges, selon les revendications 1 à 3, pour la protection, la stabilisation ou la performance améliorée de "microarrays" d'ADN ou d'ARN.

14. Utilisation de mannosyl(1-2)glucosyl(1-2)glycérat ou de leurs mélanges, selon les revendications 1 à 3, en tant qu'un additif pour des produits cosmétiques pour améliorer des propriétés d'hydratation, pour stabiliser des composés ou des liposomes, ou en tant qu'un suppresseur de radicaux livres.

15. Utilisation de mannosyl(1-2)glucosyl(1-2)glycérat ou de leurs mélanges, selon les revendications 1 à 3, pour la protection contre les dommages causés par lyophilisation, dessiccation, stress de température et la congelation sur des cellules microbiennes.

16. Utilisation de mannosyl(1-2)glucosyl(1-2)glycérat ou de leurs mélanges, selon les revendications 1 et 4 à 15, le composé étant un composant dans une formulation convenable pour protéger et/ou stabiliser des enzymes ou d'autres composants cellulaires et des biomatériaux dans des buts commerciaux, biologiques, de recherche et industriels.
